# EUROPEAN PATENT SPECIFICATION

(11) **EP 3 187 582 B1**
(45) Date of publication and mention of the grant of the patent: **23.07.2025**
(21) Application number: 15836159.2
(22) Date of filing: 26.08.2015
(51) Int. Cl.: A23C 9/12, A23C 21/02, A23C 9/142, C12N 9/96

(54) **LACTASE SOLUTION**
LAKTASELÖSUNG
SOLUTION DE LACTASE

(30) Priority: 27.08.2014 JP 2014173068
(43) Date of publication of application: 05.07.2017
(73) Proprietor: Godo Shusei Co., Ltd., Tokyo 104-8162 (JP)
(72) Inventor: OGASAWARA, Junki, Matsudo-shi Chiba 271-0064 (JP); HORIGUCHI, Hirofumi, Matsudo-shi Chiba 271-0064 (JP)
(74) Representative: Hoffmann Eitle
(86) International application number: PCT/JP2015/074121
(87) International publication number: WO 2016/031885

(56) References cited:
- EP-A1- 1 203 774
- EP-A2- 0 636 319
- WO-A1-2012/134987
- JP-A- 2004 534 527
- JP-A- H01 104 198
- JP-A- H02 100 693
- JP-A- H02 303 486
- JP-A- H06 217 797
- US-A1- 2003 230 532
- US-A1- 2010 190 965
- YAN MINGKUI ET AL: "Effects of metal ions and surfactants on the activity of transglycosylating beta-galactosidase", CHINA BREWING, August 2010 (2010-08-01), Tsinghua Tongfang Knowledge Network Technology Co., Ltd.(Beijing), Retrieved from the Internet <URL:http://en.cnki.com.cn/Article_en/CJFDTotal-ZNGZ201008031.htm> [retrieved on 20200417]
- TAKAO HATTORI ET AL.: "Kaimen Kasseizai Yoekichu deno beta-Galactosidase ni yoru To Ten'i Hanno", JAPAN SOCIETY FOR BIOSCIENCE, BIOTECHNOLOGY, AND AGROCHEMISTRY TAIKAI KOEN YOSHISHU, vol. 64, no. 3, 1990, pages 359, XP009500566

## Description

### Technical Field

The present invention relates to a lactase solution.

### Background Art

Lactose intolerance indicates a condition exhibiting various symptoms such as abdominal pain and diarrhea caused by lactose present in a food product such as a dairy product when there is congenital insufficiency of decomposing lactose. Lactose is a disaccharide consisting of galactose and glucose. To cope with lactose intolerance, decomposing in advance the lactose contained in milk or the like to galactose and glucose using the lactase enzyme is carried out in the food manufacturing industry.

A lactase solution which is used for decomposing lactose contained in milk or the like is conventionally produced as follows. After culturing lactase-producing microorganisms, lactase is extracted from cells or collected from extracellularly secreted solution , and is purified by removing impurities originating from the culture, and is sterilized by filtration after adding a stabilizer, thereby producing the lactase solution.

The lactase solution produced as above is stored, sold, and transported under a refrigerated condition (10°C or lower). After that, the lactase solution is added to milk or a dairy product by a user. As for a method of adding lactase, there are two main methods, that is, a method of adding it before sterilization of milk or the like and a method of adding it after sterilization. In the case of the former, a step for removing microorganisms by filtration is not necessarily required. However, in the case of the latter, a step for sterilizing the lactase by filtration is required. Milk obtained after addition of lactase is filled in a container and sold as a commercial product.

With regard to a production process of adding lactase to milk after sterilization as described above, it has been known that a lactase solution easily causes filter clogging during a filter sterilization step, and this is the reason of having a significant decrease in working efficiency. To cope with such a problem, for example, Patent Literature 1 (JP 6-73454 B2) describes that before deterioration products of proteins and polysaccharides as a cause of clogging are formed, the lactase solution is sterilized by filtration immediately after collecting and purifying the lactase solution. Although this method can be carried out at the time of producing the lactase solution, it cannot be carried out once the deterioration products are formed. That is because, when the lactose solution prepared by this method is filtered after storage or transport, there is a case of having an occurrence of clogging. For example, after the lactase which has been produced by the method is transported to a user, and the lactase is subjected to a filter sterilization step so that a user can add the lactase to milk or the like, an occurrence of filter clogging may be yielded. When clogging occurs, there is a problem in that the production step needs to be stopped and the filter needs to be changed, and thus a significant decrease in working efficiency is caused. When an individual production step for producing milk is continuously present, in particular, it is not possible to stop the filter sterilization step only, and thus it becomes necessary to stop entire steps for producing milk. As a result, the milk production efficiency is significantly lowered, and thus an improvement is required in this regard.

Accordingly, the method described in Patent Literature 1 is not a solution to the problem with regard to clogging during filtering of the lactase solution which has been prepared as a commercial product.

Furthermore, Patent Literature 2 (JP 2004-534527 A) describes that maintaining the concentration of polysaccharides and oligosaccharides contained in a lactase solution at a certain value or lower, and in particular, removing these materials by chromatography. However, even when the concentration of polysaccharides and oligosaccharides is kept at a certain value or lower, similarly, there is a case in which the clogging problem associated with filtering after the lactase solution has been prepared as a commercial product still occurs in some cases. Patent Literature 3 (WO 2012/134987 A1) describes a method for reducing fouling of ultrafiltration membranes used for removing virus particles from aqueous solutions.

Therefore, the above problems are not fully solved yet.

### Citation List

### Patent Literature

Patent Literature 1: JP 6-73454 B2
Patent Literature 2: JP 2004-534527 A
Patent Literature 3: WO 2012/134987 A1

### Summary of Invention

### Technical Problem

An object of the present invention is to provide a method for reducing clogging of a filtering device (filter) during a filtering step prior to addition of a previously formulated lactase solution to milk for use, and a lactase solution which is less prone to causing clogging of a filtering device.

### Solution to Problem

The present inventors have found that the clogging phenomenon is caused and also accelerated by physical stimulation such as stirring or shaking and/or temperature load. It has been further found that the clogging phenomenon can be prevented by reducing an interaction between the materials included in the lactase solution, and the present invention is completed accordingly.

Thus, the present invention provides a lactase solution according to claim 1. Advantageous embodiments are subject of the dependent claims.

### Advantageous Effects of Invention

According to the present invention, it is possible to solve the problem of filter clogging during the filtering step before adding a lactase solution to milk for use. Accordingly, filter exchange caused by clogging and frequency of sterilization or the like of a production line accompanying the filter exchange is reduced, and thus the working efficiency can be greatly enhanced. The present invention is also excellent in that, compared to a method of prior art based on removal of clogging materials or the like, it is more convenient and has no regeneration of clogging materials during storage of the lactase solution.

### Brief Description of Drawings

Fig. 1 is a drawing illustrating the result of determining the cause of having a clogging phenomenon. Panel A, panel B, and panel C each represents the result of measuring the filter permeability after shaking or standing at 10°C, 20°C, and 30°C. Panel D represents the result of measuring the lactase activity after shaking or standing at each temperature.
Fig. 2 is a drawing illustrating the result of reproducing a clogging phenomenon by stirring for 16 hours at 10°C.
Fig. 3 illustrates the result of determining the effectiveness of adding various surfactants for preventing a clogging phenomenon. In Fig. 3, ◆ represents an addition-free sample, and samples added with an additive are represented by **•**, ×, or - in which the additive is Tween80, TritonX (Tx)-100, and RYOTO polyglyester, respectively.
Fig. 4 illustrates the result of determining the effectiveness of adding various polyethylene glycols (PEG200, PEG400, PEG4000, PEG6000, PEG20000) for preventing a clogging phenomenon. In Fig. 4, ◆ represents an addition-free sample, and samples added with various polyethylene glycols are represented by **•**, ∘, **▲**, and △, respectively.
Fig. 5 illustrates the result of determining the effectiveness for preventing a clogging phenomenon as the addition concentration of TritonX-100 (panel A) and Tween80 (panel B) is changed.
Fig. 6 illustrates the result of determining the effectiveness for preventing a clogging phenomenon as the addition concentration of PEG6000 (panel A) and PEG20000 (panel B) is changed.
Fig. 7 illustrates the result of determining the effectiveness for preventing a clogging phenomenon as the addition concentration of Mg salt is changed.

### Mode for Carrying Out the Invention

The lactase solution according to the present invention essentially contains a lactase and an aggregation inhibitor for preventing aggregation of the lactase and other proteins contained in the lactase solution, and optionally contains additives such as a stabilizer. Hereinbelow, descriptions are given in the following order - (1) constitutional components of a lactase solution, (2) composition of a lactase solution (in particular, concentration of an aggregation inhibitor), (3) properties of a lactase solution, (4) method for preparing a lactase solution, and (5) method of using a lactase solution and use of a lactase solution.

### <<Constitutional components of lactase solution>>

### <Lactase>

### (Type of raw organisms)

Lactase has been isolated from a broad range of organisms including microorganisms. In many cases, the lactase is an intracellular or extracellular component of a microorganism such as *Kluyveromyces* or *Bacillus. Kluyveromyces,* in particular, *K.fragilis, K.lactis,* and the yeast of genus *Candida,* genus *Torula,* genus of *Torulopsis* or the like are a common source of yeast enzyme lactase, and *B.coagulans* or *B. circulans* is a well known source of microbial lactase. Several kinds of a lactase preparation derived from those organisms are commercially obtainable. As all of those lactases have optimum pH of pH = 6 to pH = 8, they are so-called a neutral lactase. Furthermore, an extracellular lactase is produced by *Aspergillus niger, Aspergillus oryzae,* or *Penicillium multicolor,* and in the specification of US 5,736,374 A, an example of such lactase produced by *Aspergillus oryzae* is described. The enzyme characteristics of lactase including optimum pH and optimum temperature vary depending on species. In general, an extracellular lactase is a so-called acidic lactase which has low optimum pH, that is, pH = 3.5 to pH = 5.0.

In the present invention, it is preferable to use a neutral lactase and an acidic lactase. It is particularly preferable to use a neutral lactase derived from genus *Kluyveromyces* and an acidic lactase derived from genus *Aspergillus.*

### (Method for producing lactase)

The lactase to be used in the present invention can be a lactase which has been recovered and purified from a microorganism by a common method of a prior art. The lactase to be used in the present invention may be any one of an intracellular lactase and an extracellular lactase. In the case of the former, the lactase is generally derived from a cytoplasm of a microorganism. As a specific method for producing a solution of intracellular lactase, there can be a method in which microorganisms are first cultured and a lactase is separated therefrom. For this method, it is necessary to disrupt a cell wall to release the enzyme from a cytoplasm. For cell lysis, a technique such as permeation of a cell wall using an organic solvent such as octanol, ultrasonic degradation, or French pressing is used. The lactase can be separated and recovered from a lysed cell by using a well known method. In the case of the latter, disruption of a cell wall is not necessary, and based on centrifuge or filtering after culture, for example, cells in the culture solution can be removed. After termination of fermentation or removal of cells, the lactase is recovered from the culture solution. In any of those cases, the lactase can be isolated and purified by a conventionally known means.

### <Aggregation inhibitor I>

The lactase solution of the present invention contains an aggregation inhibitor for preventing aggregation of a lactase and other proteins contained in the lactase solution, wherein the aggregation inhibitor is at least one selected from a surfactant with HLB of 12 to 15, polyethylene glycol, and a Mg ion or a salt thereof. The aggregation inhibitor which is used in the present invention can be selected to be a (Type 1) surfactant with HLB of 12 to 15. In addition, there are mentioned a (Type 2) lipotropic surfactant, (Type 3) non-ionic lipotropic surfactant, (Type 4) non-ionic surfactant, and (Type 5) natural product-based surfactant. Preferably, it is a non-ionic surfactant with HLB of 12 to 15. It is understood in this regard that, as those aggregation inhibitors are present in a system, the hydrophobic interaction between proteins is either inhibited or prevented, and as a result, forming of a clogging material caused by aggregation is prevented even under stirring or shaking for a long period of time. From the viewpoint of emulsion stability and degree of the effect of dispersing a hydrophobic substance in an aqueous solution, a surfactant with HLB of 12 to 15 is used. Hereinbelow, those surfactants are described in detail. Meanwhile, the types are classified depending on physical properties, origin, or the like, and a component belonging to one type may also belong to other type. Furthermore, it is also possible that plural kinds of surfactants of the same type may be used in combination, or plural kinds of surfactants of the different type may be used in combination.

### (Type 1)

First, "HLB" of the surfactant with HLB of 12 to 15 is a value which is calculated by Griffin method. Furthermore, more preferable HLB is 13 to 15. As for the surfactant of Type 1, a below-mentioned non-ionic surfactant can be also used in addition to an anionic surfactant and an amphoteric surfactant.

### (Type 2)

Next, examples of the lipotropic surfactant include a reaction product between alcohol, polyoxyethylene alkyl ether, fatty acid, bile acid, glycerin fatty acid ester, acetylated glycerin fatty acid ester, lower alcohol fatty acid ester, polyethylene glycol fatty acid ester, polyethylene glycol glycerin fatty acid ester, polypropylene glycol fatty acid ester, sucrose fatty acid ester, polyethylene fatty acid ester, polyoxyethylene glyceride, a lactic acid derivative of mono/diglyceride, propylene glycol diglyceride, sorbitan fatty acid ester, polyoxyethylene sorbitan fatty acid ester, a polyoxyethylene - polyoxypropylene block copolymer, transesterified vegetable oil, sterol, a sterol derivative, sugar ester, sugar ether, sucroglyceride, polyoxyethylene vegetable oil, polyoxyethylene hydrogenated vegetable oil, or polyhydric alcohol, and at least one from a group consisting of fatty acid, glyceride, vegetable oil, hydrogenated vegetable oil, and sterol, and a mixture thereof.

### (Type 3)

Furthermore, examples of the non-ionic lipotropic surfactant include a reaction product between alkyl glucoside, alkyl maltoside, alkylthioglcoside, lauryl macrogol glyceride, polyoxyethylene alkyl ether, polyoxyethylene alkyl phenol, polyethylene glycol fatty acid ester, polyethylene glycol glycerin fatty acid ester, polyoxyethylene sorbitan fatty acid ester, a polyoxyethylene-polyoxypropylene block copolymer, polyglycerin fatty acid ester, polyoxyethylene glyceride, polyoxyethylene sterol, a derivative and a homologue thereof, polyoxyethylene vegetable oil, polyoxyethylene hydrogenated vegetable oil, or polyhydric alcohol, and at least one from a group consisting of fatty acid, glyceride, vegetable oil, hydrogenated vegetable oil, and sterol, tocopherol polyethylene glycol succinate, sugar ester, sugar ether, sucroglyceride, and a mixture thereof.

### (Type 4)

Furthermore, examples of the non-ionic surfactant include n-alkyl PEO monoether such as Brij35 or polyoxyethylene (20) cetyl ether, n-alkyl phenyl PEO such as Lubrol PX, Lubrol WX, nonidet P-40, octylphenol poly(ethylene glycol ether)n10 or octylphenol poly(ethylene glycol ether)n7, tetramethyl butylphenyl PEO, n-octylglycoside, octyl-thioglycopyranoside, tween-85, tween-80, tween-65, tween-60, tween-40, tween-20, alkylaryl polyether alcohol (TritonX-100), and polyethylene glycol tert-octyl phenyl ether (TritonX-114).

### (Type 5)

Furthermore, examples of the natural product-derived surfactant include sucrose fatty acid ester, polyethylene fatty acid ester, lecithin, lysolecithin, and Quillaja saponin.

Among them, the most preferred aggregation inhibitor I is at least one selected from a group consisting of polyoxyethylene sorbitan fatty acid ester (for example, Polysorbate 80), alkylaryl polyether alcohol (for example, TritonX-100), and glycerin fatty acid ester.

### <Aggregation inhibitor II>

As an aggregation inhibitor for preventing aggregation of a lactase and other proteins contained in the lactase solution of the present invention, a protective agent having an activity of covering a surface of a lactase and other proteins may be used. As a protective agent, a polyethylene glycol is used.

Due to the reason of effective protection of proteins by having a high hydration property and a very flexible structural skeleton, polyethylene glycol is used. Number average molecular weight of polyethylene glycol is preferably 200 to 4000000, and more preferably 400 to 20000.

It is understood that, because polyether also has a hydrophilic part and a hydrophobic part, the hydrophobic interaction between a lactase and other proteins contained in the lactase solution can be prevented by it, and as a result, it has an effect of lowering the forming of clogging materials. Furthermore, it is also possible that plural kinds of polyether of the same type may be used in combination, or plural kinds of polyether of the different type may be used in combination.

As for the thickening polysaccharides which may be used include gellan gum, carageenan, xanthan gum, locust bean gum, guar gum, pectin, agar, gelatin, gum Arabic, glucomanna, tara gum, pulluran, polysaccharides of tamarind seed, tragacanth gum, karaya gum, alginic acid, sodium alginate, macrophomopsis gum, starch such as corn starch and tapioca starch, dextrin, and cyclodextrin.

### <Aggregation inhibitor III>

Furthermore, as an aggregation inhibitor for preventing aggregation of a lactase and other proteins contained in the lactase solution, a metal ion or a salt thereof which has a salt-dissolving effect can be mentioned. From the viewpoint of easily obtaining suitable ionic strength of a lactase solution, a Mg ion, or a salt thereof is used.

By adding a metal ion or a salt thereof, suitable ionic strength of the solution is obtained, and thus the hydrophobic protein interaction between a lactase and other proteins contained in the lactase solution is lowered. As a result, it is understood that, as the proteins are not likely to aggregate, it has an effect of lowering the forming of clogging materials. Furthermore, it is also possible that plural kinds of a metal ion of the same type or a salt thereof may be used in combination, or plural kinds of a metal ion of the different type or a salt thereof may be used in combination.

Those aggregation inhibitors I to III may be used singly, or the aggregation inhibitors of different type may be used in combination. For example, it is also possible to use in combination the aggregation inhibitors I and II, the aggregation inhibitors II and III, the aggregation inhibitors I and III, and the aggregation inhibitors I, II and III. Furthermore, as the effect is minor with the aggregation inhibitor III alone, it is preferable to use in combination the aggregation inhibitors I and III, and the aggregation inhibitors II and III.

### <Optional components>

The lactase solution of the present invention may contain various components, if necessary. Specific examples thereof include metal salts, various saccharides, ascorbic acid, glycerin, or the like which contribute to stabilization of a lactase, starch and dextrin as a vehicle for easy use, and inorganic salts having a buffering activity.

### <<Composition of lactase solution (in particular, amount of aggregation inhibitor)>>

### <Aggregation inhibitor I>

The aggregation inhibitor I is added within a range of 0.001% by mass to 5% by mass, preferably 0.01% by mass to 1% by mass, and more preferably 0.1% by mass to 0.5% by mass on the basis of the total mass of the lactase solution.

### <Aggregation inhibitor II>

The aggregation inhibitor II is added within a range of 0.3% by mass to 15% by mass, preferably 0.3% by mass to 10% by mass, and more preferably 0.5% by mass to 5% by mass on the basis of the total mass of the lactase solution.

### <Aggregation inhibitor III>

The aggregation inhibitor III has a concentration of 1 mM or more and 20 mM or more less, preferably 1 mM or more and 15 mM or more less, and more preferably 1 mM or more and 10 mM or less as a metal component in the lactase solution. It most preferably has the concentration of 1 mM or more and 5 mM or less. In the order of having higher salt-dissolving effect, a magnesium ion is used.

### <<Properties of lactase solution>>

### <Lactase activity>

The lactase solution of the present invention preferably has a neutral lactase activity of 10 to 100,000 NLU/g. More preferably, it has the activity of 100 to 90,000 NLU/g, and even more preferably it has the activity of 1,000 to 80,000 NLU/g. "NLU" represents Neutral Lactase Unit. A method for measuring the activity is as described below. The measurement is carried out based on hydrolysis of a substrate o-nitrophenyl-β-galactopyranoside (ONPG) to o-nitrophenyl and galactose. The reaction is terminated by addition of sodium carbonate. Thus formed o-nitrophenyl exhibits yellow color in an alkali medium, and a change in absorbance is used for the measurement of enzyme activity (expressed in NLU/g). This order is disclosed in Food Chemicals Codex (FCC) Fourth Edition, effective July 1, 1996, Committee on Food Chemicals Codex pages 801 to 802/lactase (neutral) (β-galactosidase) activity.

The lactase solution of the present invention preferably has an acidic lactase activity of 10 to 100,000 ALU/g. More preferably, it has the activity of 100 to 90,000 ALU/g, and even more preferably it has the activity of 1,000 to 80,000 ALU/g. "ALU" represents Acid Lactase Unit. A method for measuring the activity is as described below. The measurement is carried out based on hydrolysis of a substrate o-nitrophenyl-β-galactopyranoside (ONPG) to o-nitrophenyl and galactose. The reaction is terminated by addition of sodium carbonate. Thus formed o-nitrophenyl exhibits yellow color in an alkali medium, and a change in absorbance is used for the measurement of enzyme activity (expressed in ALU/g). This order is disclosed in Food Chemicals Codex (FCC) Fourth Edition, effective July 1, 1996, Committee on Food Chemicals Codex pages 802 to 803/lactase (acidic) (β-galactosidase) activity.

The lactase solution of the present invention may be any one of a neutral lactase solution and an acidic lactase solution, and it may be also a lactase solution in which both are mixed to function in a neutral to acidic condition.

### <Permeation rate after stirring (relative value)>

The determination can be made by subjecting the lactase solution of the present invention to a stirring treatment at 100 rpm, 10°C for 16 hours with a concentration for having the activity of 5,000 NLU/g or 10,000 ALU/g and calculating a decrease amount of the permeation rate at the time of terminating the permeation test (that is, permeation of 180 g) when the permeation rate (Flux) immediately after starting the permeation test at the following conditions (that is, permeation of 20 g) is 100%. It is more preferable to have a higher permeation rate at the time of terminating the permeation test. It is preferably 10% or more, more preferably 20% or more, and even more preferably 30% or more. The upper limit is 75%, for example.

Activity of the lactase solution can be adjusted by dilution with ion exchange water or concentration by ultrafiltration.

### <Permeation rate after stirring (absolute value)>

The lactase solution of the present invention is characterized in that the filter permeation rate which is measured at the following conditions is 5 kg/min × m² or more after the lactase solution is subjected to a stirring treatment at 100 rpm, 10°C for 16 hours with a concentration for having the activity of 5,000 NLU/g or 10,000 ALU/g. The filter permeation rate is preferably 8 kg/min × m² or more, more preferably 10 kg/min × m² or more, and most preferably 20 kg/min × m² or more.

### <Conditions for reproducing clogging phenomenon>

The filter clogging phenomenon of a lactase can be reproduced by carrying out a reciprocal shaking or stirring operation of a lactase solution. Time for carrying out a reciprocal shaking or stirring operation can be set between 30 minutes and 7 days, although it may vary depending on the heating temperature and activity value of a lactase solution. It is preferably between 1 hour to 6 days, and more preferably between 2 hours and 5 days. By carrying out heating of a lactase solution while performing a reciprocal shaking or stirring operation, a clogging phenomenon can be promoted.

The reciprocal shaking of a lactase solution is preferably 10 spm (strokes/minute) to 300 spm (strokes/minute), more preferably 20 spm (strokes/minute) to 250 spm (strokes/minute), and even more preferably 30 spm (strokes/minute) to 200 spm (strokes/minute).

The stirring operation of a lactase solution is preferably 10 rpm (rotation/minute) to 300 rpm (rotation/minute), more preferably 20 rpm (rotation/minute) to 250 rpm (rotation/minute), and even more preferably 30 rpm (rotation/minute) to 200 rpm (rotation/minute).

Temperature for heating a lactase solution is preferably 5°C to 70°C or so, more preferably 6°C to 65°C, and even more preferably 7°C to 60°C. As the temperature increases, it is easier to have an occurrence of a clogging phenomenon.

It is sufficient that the lactase activity of a lactase solution is within a range of 10 to 100,000 NLU/g, or a range of 10 to 100,000 ALU/g. As the activity value increases, it is easier to have an occurrence of a clogging phenomenon.

### <Filter permeability test 1>

The filter permeability can be evaluated, in a low temperature environment, by having a sample which has been adjusted to have lactase content to maintain a constant activity permeated through a certain filter with certain membrane area under pressure, and measuring the rate of the permeation. Specifically, the evaluation can be carried out as described below.

### (Order for measuring filter permeability)

The following operation is carried out in a low temperature to room temperature (for example, 1 to 30°C, preferably 4 to 15°C, and more preferably 8 to 12°C) environment.

### 1. Temperature of measurement device or the like

The measurement device, lactase solution sample, and ion exchange water are cooled to low temperature to room temperature (for example, 1 to 30°C, preferably 4 to 15°C, and more preferably 8 to 12°C).

### 2. Preparation of lactase solution

The lactase activity of a lactase solution sample is adjusted to a constant level by dilution with ion exchange water or concentration by ultrafiltration followed by thorough mixing. Meanwhile, the lactase activity may be adjusted to any one of 10 to 2,000 NLU/g and 10 to 4,000 ALU/g or so depending on the viscosity of a lactase solution.

### 3. Configuration of test device

As for the test device, a filter holder mounted with a pressurizable tank is used as an inlet for measurement sample, and an additional separate filter holder may be connected thereto and used. The filter holder mounted with a pressurizable tank can be used by itself. However, to have a condition for allowing clogging of a filter, the former is preferable, and the diameter (ϕ) thereof is preferably larger than the ϕ of a filter holder to be connected.

In general, the sample permeation part in a filter holder has a configuration in which, from the opening side of a measurement sample, an O-ring, a membrane, and a support screen are provided, but it is not limited thereto. The membrane has, although not particularly limited, pore diameter of preferably 0.05 to 0.50 µm, and more preferably 0.1 to 0.45 µm. Furthermore, it may be hydrophilic or hydrophobic. More preferably, it is hydrophilic. ϕ is not particularly limited as long as the effective membrane area can be adjusted with it. The effective membrane area can be adjusted by sealing with a liquid-impermeable label or the like, and it is 0.5 to 1.8 cm², for example, and preferably 1.0 to 1.5 cm².
4. The lactase solution sample which has been diluted in the above 2 (hereinbelow, abbreviated as 'sample') is introduced to an inlet for measurement sample.
5. By using nitrogen gas (air, oxygen, argon, or the like may be also used) and applying pressure of 0.1 to 1 MPa, and preferably 0.2 MPa to a filter holder mounted with a pressurizable tank, elapsed time is recorded according to addition of a sample in certain amount (for example, 10 to 30 g), and preferably 20 g.

### (Determination of clogging inhibiting effect)

When a lactase solution of a prior art (that is, not containing an aggregation inhibitor) is used as a lactase solution according to the above order of measuring filter permeability, the permeation rate is greatly lowered at the time of terminating the permeation test. If the permeation rate is higher than the permeation rate of a lactase solution of a related art, it can be determined that the substance added as an aggregation inhibitor has a clogging inhibiting effect.

### <<Method for preparing lactase solution>>

The specific method for preparing a lactase of the present invention is as described above. Although the descriptions are omitted herein, the method includes (1) a step of extracting a lactase accompanied with disruption of a cell wall after performing yeast culture, and (2) a purification step for removing impurities derived from a culture product from the extracted lactase. The method for preparing a lactase solution of the present invention includes (3) a step of adding an aggregation inhibitor (+ other additives, if necessary) to the above lactase (which may be freshly prepared product or a commercially available product), and (4) a step of filtering for sterilization.

### <<Method of using lactase solution and use of lactase solution>>

### (Method of using lactase solution)

With regard to a specific mode of using the lactase solution, the lactase solution is used for production of fermented milk, for example. Examples of a method for producing fermented milk based on decomposition of lactose include the following:
1. lactose is decomposed by adding lactase to milk before sterilization, and then the lactase is inactivated simultaneously with thermal sterilization of milk followed by fermentation of milk (JP 5-501197 A),
2. lactase is added to sterilized milk for decomposition of lactose, and then the lactase is inactivated by a heating treatment followed by fermentation of milk,
3. lactose in milk is decomposed using immobilized lactase followed by fermentation of milk (JP 46-105593 A and JP 59-162833 A), and
4. raw materials obtained in advance by decomposition or removal of lactose are used as sterilized milk for fermentation.

Furthermore, as a specific mode of using the lactase solution of the present invention, the lactase solution can be used for production of long-life milk. Long-life milk is milk for long term storage, and the process for producing it includes a sterilization step and a continuous aseptic packaging step. In general, milk is treated by ultra-high temperature short-time sterilization at 135 to 150°C for several seconds, and filled by a step by which a paper pack sterilized in advance with hydrogen peroxide is aseptically packaged.

The lactase solution added to long-life milk is generally added after filtering sterilization when milk after ultra-high temperature short-time sterilization is filled.

### (Use of lactase solution)

The lactase solution according to the present invention is particularly suitable for use in production of a dairy product. As described herein, the dairy product indicates ice, milk products such as long-life milk, yoghurt, raw cream, sour cream, cheese, or the like. The lactase solution of the present invention is particularly useful for production of long-life milk.

### (Use and pH profile of lactase)

When the use of a lactase is considered, two main categories are present depending on whether the lactase is a neutral lactase or an acidic lactase. That is due to the pH profile of each use. It can be said that, for a use in neutral pH, a neutral lactase is generally preferable while an acidic lactase is more suitable for a use in an acidic range.

Herein, the aforementioned filter permeability test 1 can be applied not only to a lactase solution but also to a protein and a peptide. That is because, as described below, an aggregated product is believed to be formed as hydrophobic parts in a molecule contact each other to yield an adhesion.

Hereinbelow, detailed descriptions are given for the filter permeability test.

The present test method is a test method for evaluating clogging characterized in that,
the test method is based on measurement of a change in permeation amount (permeate (kg/m²)) and/or permeation rate (flux (kg/min × m²)) of a liquid per unit area, in which the method uses a unit having a first cylindrical flow path with first diameter, a second cylindrical flow path with second diameter that is smaller than the above first diameter, in which the second cylindrical flow path is connected to the first cylindrical flow path for allowing fluid communication and disposed at a downstream of the first cylindrical flow path, and a membrane filter disposed on an opening of the second cylindrical flow path, which is present on the opposite side of the first cylindrical flow path,
the measurement method has a step of pressurizing a liquid as a measurement subject and discharging the liquid, through the first cylindrical flow path and the second cylindrical flow path, from the membrane filter to outside of the unit, and
the unit has a support screen disposed on a downstream side of the membrane filter and also on a site close to the membrane filter, and, as one or plural sealing members attached on a filter side of the membrane, a liquid impermeable sealing member on the support screen in which total area of the one or plural sealing members is smaller than the area of the membrane filter and the membrane filter is in contact with the liquid impermeable sealing member.

According to the test method of the present invention, a phenomenon of clogging of a membrane filter can be reproduced so that a determination test regarding solving means or the like can be briefly carried out.

### Examples

### 1. Determination of conditions for reproducing clogging phenomenon

By using YNL (manufactured by GODO SHUSEI CO., LTD., product name: GODO-YNL) as a lactase solution, conditions for having clogging were investigated. Meanwhile, GODO-YNL which has been used is a neutral lactase derived from *Kluyveromyces,* and it has the activity of 5,000 NLU/g.

### (Reciprocal shaking treatment)

200 g of YNL was added to a 500 ml Aiboi (wide mouth (manufactured by AS ONE Corporation., product number of 7-2102-03)), and subjected to reciprocal shaking (100 spm (strokes/minute)) or standing for 0, 2, 4, or 7 days at 10°C, 20°C, or 30°C by using a shaking device (manufactured by Taiyo Kagaku Co., Ltd., product name: TAIYO INCUBATOR PERSONAL, stroke of 3 cm). Each YNL was weighed in an amount of 40 g, and then by using a sample of which activity is adjusted to 1,000 NLU/g according to dilution of 5 times with ion exchange water, the filter permeability was measured.

### (Filter permeability test)

Detailed conditions of the filter permeability test 1 for the present example are described in detail hereinbelow.

### (Procedure for measuring filter permeability)

The following operations were performed in an environment of 10°C.
1. The measuring device, lactase solution sample, and ion exchange water were cooled to 10°C.
2. The lactase solution sample was adjusted to have lactase activity of 1,000 NLU/g by dilution with ion exchanged water or concentration by ultrafiltration followed by thorough mixing.
3. For a test, a stainless holder mounted with a 47 mm tank (manufactured by Advantec Toyo Kaisha, Ltd., product name of "KST-47"), which is connected with a 25 mm stainless steel filter holder (manufactured by Pall Corporation, product number of 1209 (effective membrane area of 3.7 cm²)), was used as a device. The sample permeation part in the above filter holder has a configuration in which, from the opening side of a measurement sample, an O-ring, a membrane, and a support screen are provided, (in the test of the present invention, a filter of the stainless holder mounted with a tank and a part of the filter holding part (that is, support screen and its supporter) are not mounted). As a membrane, DURAPORE (product name, pore diameter of 0.22 µm, ϕ 25 mm, made of hydrophilic PVDF) manufactured by Merck Millipore was used. As a support screen, Type 316 stainless steel belonging to the above filter holder was used. Furthermore, to control the permeation rate, on top part of the support screen (that is, on the opening side of a measurement sample), 4 pieces of a circular label seal with diameter of 0.9 cm (manufactured by AONE, product name of AONE color label 07010) were attached such that they have left and right symmetry (effective membrane area of 1.26 cm²) followed by setting of a membrane filter (pore diameter of 0.22 µm). The membrane was rinsed with 50% aqueous solution of glycerin and then mounted on the stainless filter holder.
4. The lactase solution sample (that is, sample) obtained by dilution in the above 2 was added to a stainless holder mounted with a tank.
5. By using nitrogen gas, pressure of 0.2 MPa was applied to the stainless holder mounted with a tank, and the elapsed time was recorded for each permeation of 20 g. From the results obtained therefrom, the permeation amount and (permeate (kg/m²)) and permeation rate (flux (kg/min × m²)), which are calculated in terms of the value per 1 m² membrane, were obtained according to the following method.

### Calculation formula (concept formula)

Permeate (kg/m2) = Permeation amount at point n (g)/(Membrane radius (mm) × Membrane radius (mm) × Circular constant) (m2) × 1000 Flux (kg/min × m2) = (Permeate at point n - Permeate at point (n-1))/(Permeation time at point n (min) - Permeation time at point (n-1) (min))
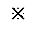 n represents a measurement point. To record the elapsed time for each permeation of 20 g, when point n indicates the permeation amount or permeate at the time of permeation of 20 g, point (n-1) indicates the permeation amount or permeate at the time of permeation of 0 g.

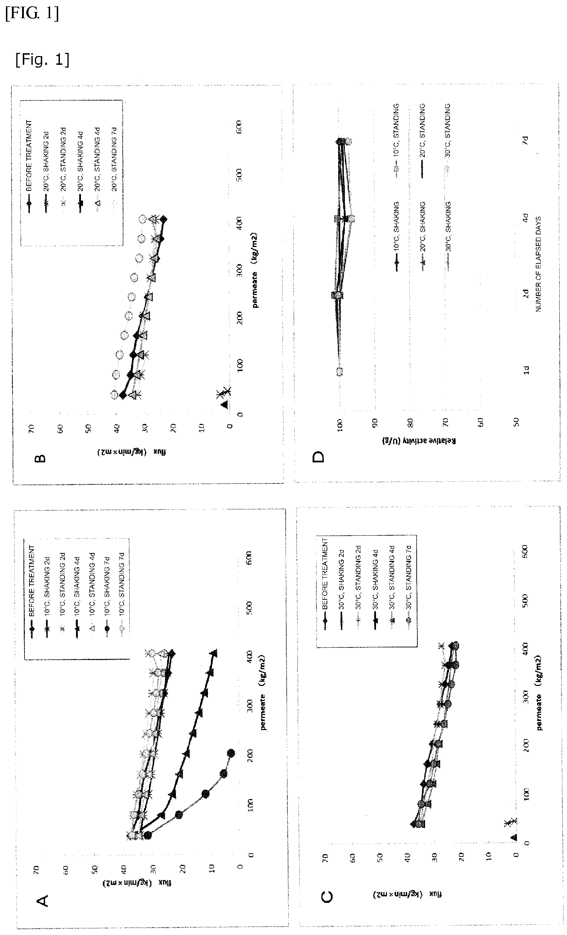

The results are shown in Fig. 1. Panel A indicates the result obtained after standing or reciprocal shaking at 10°C, panel B indicates the result at 20°C, and panel C indicates the result at 30°C. At each temperature, all the samples which have been allowed to stand exhibited a small decrease in the permeation rate regardless of the days of standing. On the other hand, the permeation amount from a sample after reciprocal shaking exhibited a dramatically reduced permeation rate in accordance with an increase of the days of shaking. The measurement cannot be made for the sample shaken for 7 days at 20°C or 30°C due to clogging. As such, it is considered that clogging has mainly occurred due to forming of an aggregate as caused by reciprocal shaking treatment. Meanwhile, the sample after reciprocal shaking at 20°C and the sample after reciprocal shaking at 30°C exhibited slight turbidity after 2 days, but no turbidity was measured from the sample which has been allowed to stand. However, in both of the sample after reciprocal shaking and the sample after standing, there was a tendency that the permeation amount decreases as the temperature increases. It was found that the increased temperature accelerates forming of an aggregate which is generated by shaking.

As shown in panel D, the lactase activity hardly changed in the case of having shaking or standing at each temperature. As such, it was found that the forming of an aggregate which causes clogging hardly has any effect on the lactase activity.

It was also investigated whether or not the same phenomenon as above is observed with stirring instead of reciprocal shaking. 50 g of YNL was added to a 100 ml beaker and subjected to stirring for 16 hours at 10°C at each stirring rate (minimum stirring rate: 100 rpm, rate 2: 170 rpm, rate 3: 240 rpm, and rate 4: 310 rpm)) by using a stirring device (manufactured by AS ONE Corporation, product name of HS-400). After that, the sample was prepared in the same manner as above, and the filter permeability was measured in the same manner as the filter permeability test above.

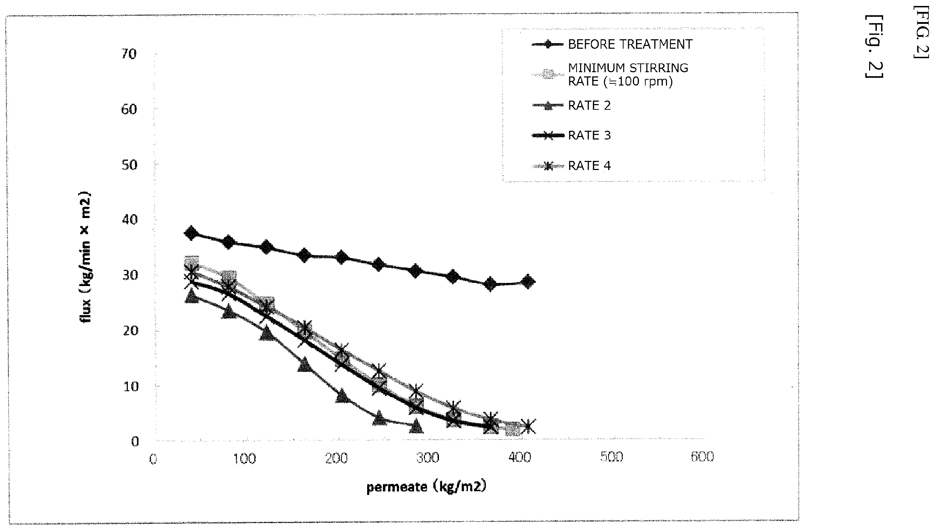

The results are shown in Fig. 2. Compared to those having no treatment, a significant deterioration in permeability was observed according to a stirring operation.

From the above results, it was found that the physical stimulation such as shaking or stirring is the main reason of forming an aggregate which yields an occurrence of clogging. As such, it is believed that an aggregate is also formed by vibration or the like which is generated during transport after production of a lactase solution.

Meanwhile, although not illustrated, when the above stirring treatment was carried out by using those in which content of polysaccharides and oligosaccharides included in a lactase solution was less than 10 g/kg, or more than 10 g/kg, and the above stirring treatment was carried out, the aggregate was also formed in any lactase solution at the same level as above. Namely, it was demonstrated that the occurrence of an aggregate is hardly affected by polysaccharides and oligosaccharides included in a lactase solution.

In the following determinations, the test was carried out while the condition for reproducing the aggregate forming to cause a clogging phenomenon includes stirring at 100 rpm, 10°C for 16 hours.

### 2. Investigation of material for inhibiting forming of aggregate

The mechanism for having an occurrence of a clogging material (that is, aggregate) caused by shaking or temperature is believed to be as follows, although it remains unclear. According to physical stimulation such as shaking, collision between a protein molecule and a protein molecule is caused. At that time, according to contact and adhesion between hydrophobic parts in the molecule, an aggregate is formed. As such, the present inventors considered the possibility of preventing, by addition of a component capable of lowering hydrophobic interactions, forming of an aggregate which causes clogging, and they investigated the effects of addition of various components.

### 2-1. Surfactant

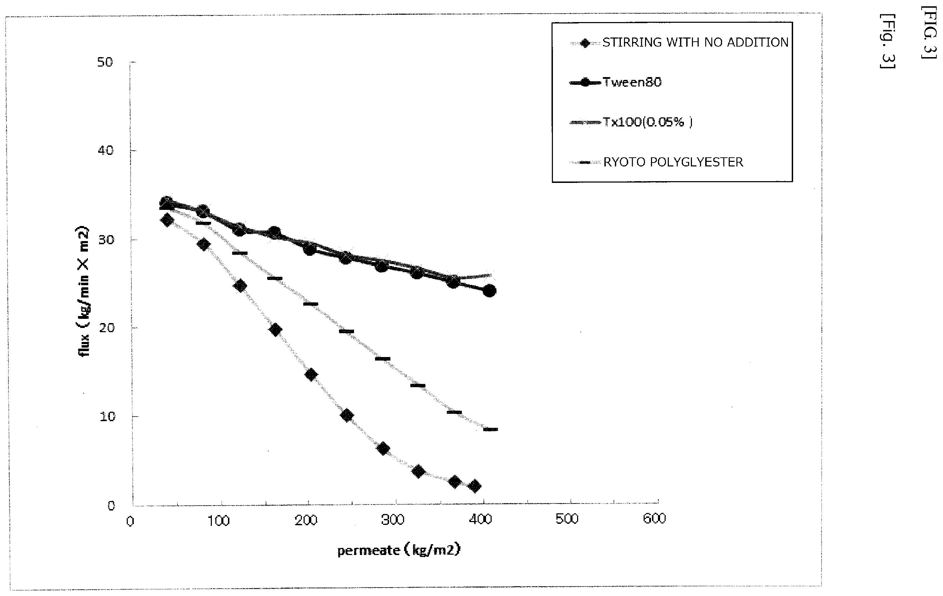

As a surfactant, Tween80 (HLB 15.0), TritonX-100 (Tx100, HLB 13.5) and RYOTO polyglyester (HLB 12.0) were added, each at a concentration of 0.1% by mass, to YNL (in 100 ml beaker, 50 g), and according to a stirring treatment, an aggregate causing a clogging phenomenon was formed. After that, a sample was prepared in the same manner as above, and the filter permeability was measured by the above filter permeability test. By using a control in which a stirring treatment has been carried out without addition of an additive, the filter permeability was also measured. The results are shown in Fig. 3. Among the added materials, TritonX-100 and Tween80 were particularly effective as an aggregation inhibitor.

Herein, specific determination of clogging was performed by calculating the decrease (in %) at the time of end of the test (that is, permeation of 180 g) (that is, relative permeation rate (%) at permeation of 180 g) compared to Flux immediately after start of the test (that is, permeation of 20 g). With regard to the determination of clogging inhibition effect of an additive, it can be found to be effective when the above calculation result is higher than the control (5.8%). Because the calculation value of a sample with no stirring was about 75%, this value was taken as the maximum value. The result of not less than 35% was evaluated as ⊚, the result of 10 to less than 35% was evaluated as ∘, the result of 0 to less than 10% was evaluated as ×, and the result showing impermeability for 200 g was evaluated as ××. The results are shown in Table 1.

### [Table 1]

**[Table 1]**

| Surface active agent | Tween80 | Tx100 | RYOTO polyglyester |
|---|---|---|---|
| Permeation rate at the time of permeation of 366 kg/m² (kg/min x m²) | 24.9 | 25.4 | 10.4 |
| Flux at the time of permeation of 20 g (kg/min x m²) | 33.9 | 34.4 | 33.5 |
| Flux at the time of permeation of 180 g (kg/min x m²) | 24.9 | 25.5 | 10.4 |
| Relative permeation rate (%) | 73.5 | 74.0 | 30.9 |
| Evaluation | ⊚ | ⊚ | ○ |

### 2-2. Polyalkylene glycol

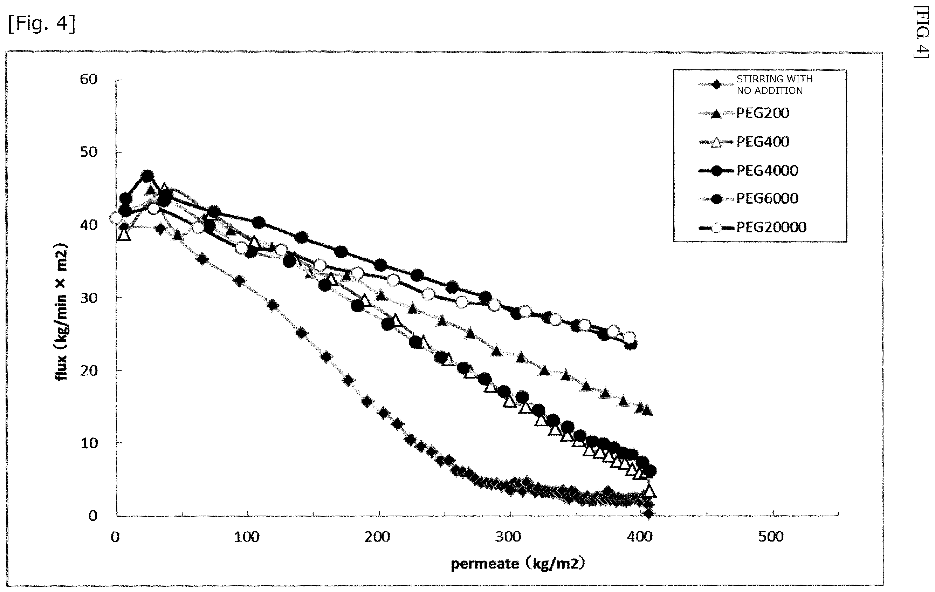

The filter permeability was measured in the same manner as the surfactant except that, as polyalkylene glycol, polyethylene glycol (PEG200 (manufactured by Wako Pure Chemical Industries, Ltd.), PEG400 (manufactured by KANTO CHEMICAL CO., INC.), PEG4000 (manufactured by KANTO CHEMICAL CO., INC.), PEG6000 (manufactured by KANTO CHEMICAL CO., INC.), and PEG200000 (manufactured by Wako Pure Chemical Industries, Ltd.)) were used at 0.5% by mass. The results are shown in Fig. 4 and Table 2.

### [Table 2]

**[Table 2]**

| Polyethylene glycol (PEG) | PEG200 | PEG400 | PEG4000 | PEG6000 | PEG20000 |
|---|---|---|---|---|---|
| Permeation rate at the time of permeation of 366 kg/m² (kg/min x m²) | 17.4 | 8.9 | 25.3 | 10.1 | 25.6 |
| Flux at the time of permeation of 20 g (kg/min x m²) | 40.8 | 44.4 | 44.3 | 42.6 | 41.9 |
| Flux at the time of permeation of 180 g (kg/min x m²) | 17.5 | 9.2 | 25.2 | 10.3 | 25.4 |
| Relative permeation rate (%) | 42.9 | 20.7 | 56.9 | 24.2 | 60.6 |
| Evaluation | ⊚ | ○ | ⊚ | ○ | ⊚ |

### 2-3. Metal ion and salt thereof

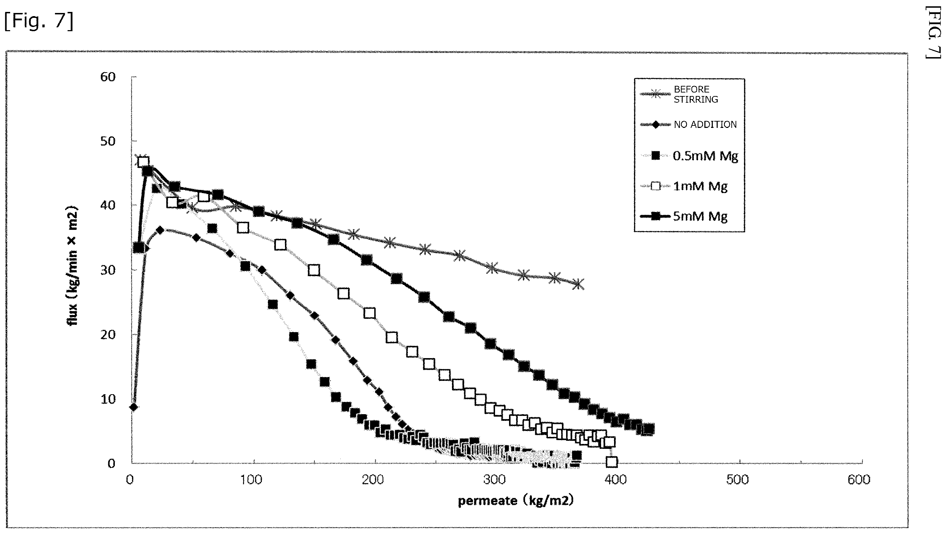

The filter permeability was measured in the same manner as the surfactant except that, as a metal salt or a salt thereof, MgSO₄ was added as a metal component to have pre-determined concentration. The results are shown in Fig. 7 and Table 6 together with the determination of the addition concentration which is described below.

### 3-1. Determination of addition concentration (surfactant)

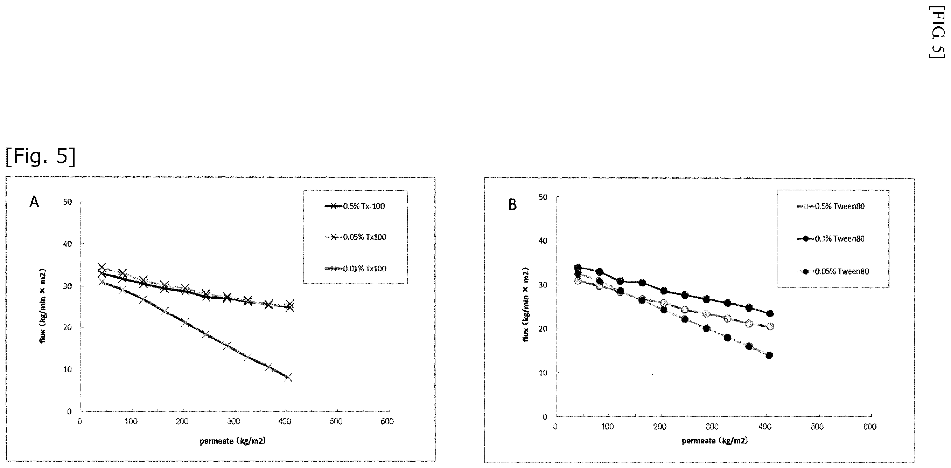

With TritonX-100 and Tween80 which were found to be particularly effective in the above test, the same test as above was carried out with modification of an addition amount to determine the effective addition concentration. The results are shown in Fig. 5 and Table 3.

### [Table 3]

**[Table 3]**

| Surface active agent (Concentration %) | Tx100 (0. 01%) | Tx100 (0.05%) | Tx100 (0. 5%) | Tween80 (0.05%) | Tween80 (0.1%) | Tween80 (0.5%) |
|---|---|---|---|---|---|---|
| Permeation rate at the time of permeation of 366 kg/m² (kg/min x m²) | 10.6 | 25.5 | 25.7 | 16.1 | 24.9 | 21.3 |
| Flux at the time of permeation of 20 g (kg/min x m²) | 30.1 | 34.4 | 33.0 | 32.6 | 33.9 | 30.9 |
| Flux at the time of permeation of 180 g (kg/min x m²) | 10.6 | 25.5 | 25.7 | 16.1 | 24.9 | 21.3 |
| Relative permeation rate (%) | 35.1 | 74.0 | 78.0 | 49.3 | 73.6 | 68.8 |
| Evaluation | ⊚ | ⊚ | ⊚ | ⊚ | ⊚ | ⊚ |

Under the conditions of this test, the effect was observed when TritonX-100 (panel A) is in a range of 0.01 to 0.5% and Tween80 (panel B) is in a range of 0.05 to 0.5%.

### 3-2. Determination of addition concentration (polyalkylene glycol)

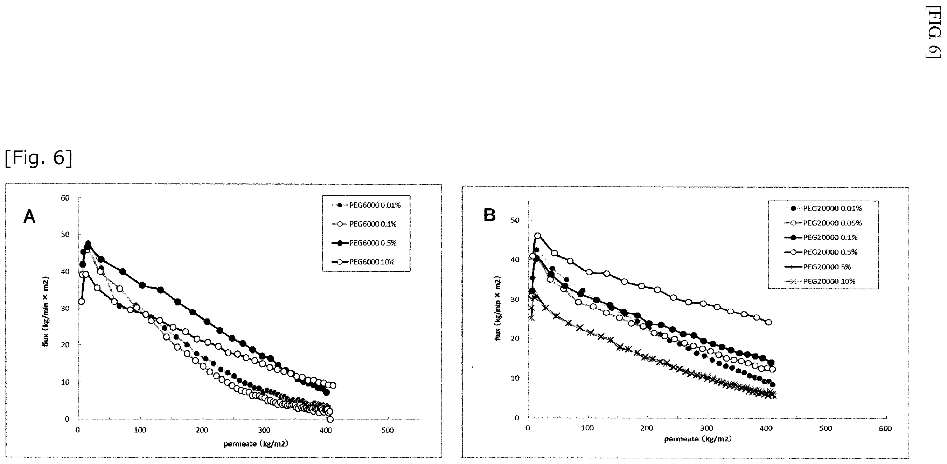

Various polyethylene glycols were used as polyalkylene glycol and, with modification of the addition concentration, the same test as the surfactant was performed and the effective addition concentration was determined. The results are shown in Fig. 6, and Table 4 and Table 5.

### [Table 4]

**[Table 4]**

| PEG6000 (Concentration %) | 0.01% | 0.50% | 10.0% |
|---|---|---|---|
| Permeation rate at the time of permeation of 366 kg/m² (kg/min x m²) | 4.4 | 10.1 | 11.0 |
| Flux at the time of permeation of 20 g (kg/min x m²) | 39.7 | 42.6 | 34.4 |
| Flux at the time of permeation of 180 g (kg/min x m²) | 4.6 | 9.9 | 10.9 |
| Relative permeation rate (%) | 11.6 | 23.2 | 31.7 |
| Evaluation | ○ | ○ | ○ |

### [Table 5]

**[Table 5]**

| PEG20000 (Concentration %) | 0.01% | 0.05% | 0.10% | 0.50% | 5.0% | 10.0% |
|---|---|---|---|---|---|---|
| Permeation rate at the time of permeation of 366 kg/m² (kg/min x m²) | 11.2 | 14.0 | 16.1 | 26.1 | 7.7 | 7.6 |
| Flux at the time of permeation of 20 g (kg/min x m²) | 37.6 | 34.5 | 35.6 | 41.9 | 26.7 | 26.4 |
| Flux at the time of permeation of 180 g (kg/min x m²) | 11.1 | 13.8 | 16.1 | 25.4 | 7.3 | 7.1 |
| Relative permeation rate (%) | 29.5 | 40.0 | 45.2 | 60.6 | 27.3 | 26.9 |
| Evaluation | ○ | ⊚ | ⊚ | ⊚ | ○ | ○ |

### 3-3. Determination of addition concentration (metal salt)

MgSO₄ was used as metal salt and, with modification of the addition concentration (0.5 mM, 1 mM, and 5 mM), the same test as the surfactant was performed and the effective addition concentration was determined. The results are shown in Fig. 7 and Table 6.

### [Table 6]

**[Table 6]**

| Metal salt (Concentration %) | Mg (0.5mM) | Mg (1mM) | Mg (5mM) |
|---|---|---|---|
| Permeation rate at the time of permeation of 366 kg/m² (kg/min x m²) | 0.8 | 4.4 | 10.5 |
| Flux at the time of permeation of 20 g (kg/min x m²) | 40.2 | 42.0 | 44.1 |
| Flux at the time of permeation of 180 g (kg/min x m²) | 0.5 | 4.4 | 9.9 |
| Relative permeation rate (%) | 1.2 | 10.4 | 22.4 |
| Evaluation | × | ○ | ○ |

Meanwhile, although not illustrated, the above stirring treatment was also carried out for a lactase solution in which content of polysaccharides and oligosaccharides was less than 10 g/kg, and a lactase solution in which the content was more than 10 g/kg and Triton X-100 was contained at 0.01%, 0.05%, and 0.5%, respectively and Tween80 was contained at 0.05%, 0.1%, and 0.5% respectively. As a result, the same tendency as shown in Table 1 and Table 2 was exhibited. In addition, even when various kinds of polyethylene glycol and Mg salt were added, the same tendency was exhibited in terms of the enhancement of the filter permeability. Namely, it is demonstrated that the Flux and the relative permeation rate are hardly affected by polysaccharides and oligosaccharides that are included in a lactase solution.

## Claims

1. A lactase solution of which a permeation rate is 5 kg/min × m² or more upon measuring at the time of permeation of 366 kg/m² through a membrane filter with a pore diameter of 0.22 µm at a concentration for having an activity of 1,000 NLU/g or 2,000 ALU/g, after being subjected to a stirring treatment at 100 rpm, 10°C for 16 hours with a concentration for having an activity of 5,000 NLU/g or 10,000 ALU/g,
wherein the lactase solution comprises an aggregation inhibitor,
wherein the aggregation inhibitor is at least one selected from a surfactant with HLB of 12 to 15, polyethylene glycol, and a Mg ion or a salt thereof, wherein a concentration of the surfactant with HLB of 12 to 15 is 0.001% by mass to 5% by mass, a concentration of the polyethylene glycol is 0.3% by mass to 15% by mass, and a concentration of the Mg ion or the salt thereof is 1 mM to 20 mM.

2. The lactase solution according to claim 1, wherein the permeation rate at the time of terminating a permeation test (that is, at the time of permeation of 180 g) is 10% or more when the permeation rate immediately after starting the permeation test (that is, at the time of permeation of 20 g) is 100%.

3. The lactase solution according to any one of the preceding claims, wherein the lactase activity is 10 to 100,000 NLU/g or 10 to 100,000 ALU/g.

4. The lactase solution according to any one of the preceding claims, which comprises glycerin.

## Patentansprüche

1. Laktaselösung, deren Permeationsrate 5 kg/min × m² oder mehr ist bei Messung bei einer Permeationszeit von 366 kg/m² durch einen Membranfilter mit einem Porendurchmesser von 0,22 µm bei einer Konzentration, um eine Aktivität von 1.000 NLU/g oder 2.000 ALU/g aufzuweisen, nachdem sie 16 Stunden lang einer Rührbehandlung bei 100 UpM und 10 °C mit einer Konzentration, um eine Aktivität von 5.000 NLU/g oder 10.000 ALU/g aufzuweisen, unterzogen wurde,
wobei die Laktaselösung einen Aggregationshemmer umfasst,
wobei der Aggregationshemmer mindestens eines ist, ausgewählt aus einem Tensid mit HLB von 12 bis 15, Polyethylenglykol und einem Mg-Ion oder einem Salz davon, wobei eine Konzentration des Tensids mit HLB von 12 bis 15 von 0,001 Masse-% bis 5 Masse-% beträgt, wobei eine Konzentration des Polyethylenglykols von 0,3 Masse-% bis 15 Masse-% beträgt und eine Konzentration des Mg-Ions oder des Salzes davon von 1 mM bis 20 mM beträgt.

2. Laktaselösung nach Anspruch 1, wobei die Permeationsrate zum Zeitpunkt der Beendigung eines Permeationstests (das heißt bei der Permeationszeit von 180 g) 10 % oder mehr beträgt, wenn die Permeationsrate unmittelbar nach Beginn des Permeationstests (das heißt bei der Permeationszeit von 20 g) 100 % beträgt.

3. Laktaselösung nach einem der vorstehenden Ansprüche, wobei die Laktaseaktivität 10 bis 100.000 NLU/g oder 10 bis 100.000 ALU/g beträgt.

4. Laktaselösung nach einem der vorstehenden Ansprüche, die Glycerin umfasst.

## Revendications

1. Solution de lactase dont un taux de perméation est 5 kg/min × m² ou plus lors d'un mesurage au moment de perméation de 366 kg/m² à travers un filtre à membrane avec un diamètre de pore de 0,22 µm à une concentration permettant de présenter une activité de 1000 NLU/g ou 2000 ALU/g, après avoir été soumise à un traitement d'agitation à 100 tr/min, 10 °C pendant 16 h avec une concentration permettant de présenter une activité de 5000 NLU/g ou 10.000 ALU/g,
dans laquelle la solution de lactase comprend un inhibiteur d'agrégation,
dans laquelle l'inhibiteur d'agrégation est au moins un sélectionné dans un tensioactif avec un HLB de 12 à 15, un polyéthylène glycol, et un ion Mg ou un sel de celui-ci, dans laquelle une concentration du tensioactif avec un HLB de 12 à 15 est 0,001 % en masse à 5 % en masse, une concentration du polyéthylène glycol est 0,3 % en masse à 15 % en masse, et une concentration de l'ion Mg ou du sel de celui-ci est 1 mM à 20 mM.

2. Solution de lactase selon la revendication 1, dans laquelle le taux de perméation au moment de terminer un essai de perméation (c'est-à-dire, au moment de perméation de 180 g) est 10 % ou plus lorsque le taux de perméation immédiatement après avoir commencé l'essai de perméation (c'est-à-dire, au moment de perméation de 20 g) est 100 %.

3. Solution de lactase selon l'une quelconque des revendications précédentes, dans laquelle l'activité de lactase est 10 NLU/g à 100.000 NLU/g ou 10 NLU/g à 100.000 ALU/g.

4. Solution de lactase selon l'une quelconque des revendications précédentes, qui comprend une glycérine.
